# EUROPEAN PATENT SPECIFICATION

(11) **EP 1 509 234 B1**
(45) Date of publication and mention of the grant of the patent: **17.12.2008**
(21) Application number: 03724064.5
(22) Date of filing: 18.04.2003
(51) Int. Cl.: A61K 31/7072, A61K 31/7076, A61K 31/708, A61P 1/00

(54) **TREATMENT OF MUCOSITIS**
BEHANDLUNG VON MUKOSITIS
TRAITEMENT DE LA MUCITE

(30) Priority: 21.05.2002 US 382225 P
(43) Date of publication of application: 02.03.2005
(73) Proprietor: ABBOTT LABORATORIES, Abbott Park IL 60064-3500 (US)
(72) Inventor: SHALWITZ, Robert, A., Bexley, OH 43209 (US); JOHNS, Paul, W., Columbus, OH 43221 (US); DAS, Tapas, Worthington, OH 43085 (US); LEACH, James, L., Columbus, OH 43202 (US)
(74) Representative: Modiano, Micaela Nadia
(86) International application number: PCT/US2003/011840
(87) International publication number: WO 2003/099297

(56) References cited:
- GIL-FERNANDEZ, G. ET AL: "Antiviral activity of uridine 5'-diphosphate glucose analogs against some enveloped viruses in cell culture" ANTIVIRAL RESEARCH (1987), 8(5-6), 299-310 , XP009015398
- GROENINGEN C J ET AL: "MODULATION OF FLUOROURACIL TOXICITY WITH URIDINE" SEMINARS IN ONCOLOGY, BETHESDA, MD, US, vol. 19, no. 2, SUPPL 3, 1 April 1992 (1992-04-01), pages 148-154, XP002083217
- PATENT ABSTRACTS OF JAPAN vol. 1996, no. 01, 31 January 1996 (1996-01-31) & JP 07 247210 A (SUNSTAR INC), 26 September 1995 (1995-09-26)

## Description

### TECHNICAL FIELD

The present invention is directed to a method for alleviating mucositis, especially stomatitis and esophagitis. Other aspects of the invention are directed to compositions for alleviating mucositis, including pharmaceutical and liquid nutritional compositions.

### BACKGROUND OF THE INVENTION

Treatments such as chemotherapy and radiotherapy can be effective at destroying tumors because it targets the most rapidly growing tissues. The mechanism involves impairment of DNA synthesis or interference with metabolic processes required for rapidly dividing cells. While tumor cells are selectively targeted by anticancer treatments, the most rapidly growing tissues of the host are also susceptible to these effects. The mucosal epithelium of the alimentary tract has one of the most rapid rates of cell division of any body tissue, and is therefore a major site of toxicity for anticancer regimens.

The linings of the mouth and esophagus are particularly sensitive to chemotherapy and radiation. The oral ulcerations characteristic of mucositis (also referred to as 'stomatitis') are a major clinical problem causing considerable pain, increased susceptibility to infection and inability to eat. Damage to the intestinal lining also commonly occurs in the small bowel, and less frequently in the large bowel, leading to severe diarrhea and pain. (Verdi C J 1993 Cancer therapy and oral mucositis. An appraisal of drug prophylaxis. Drug Safety 9:185-195; Sonis S T 1993 Oral complications of cancer chemotherapy In VT DeVita Jr., S Hellman and S A Rosenberg (ed) Cancer, Principles and Practice of Oncology, pp 2385-2394. Philadelphia, J B Lippencott Co).

In general, mucositis appears within 5 to 10 days of the drug or radiation treatment and can last several weeks. The severity of mucositis can limit subsequent doses of chemotherapy or radiation. Patients suffering mucositis may need several weeks, or more, of intravenous feeding as a result of the mouth ulcers, cramps, extreme pain, gut denuding, and severe diarrhea (Verdi 1993; Sonis 1993).

About 40% of all patients receiving chemotherapy develop significant mucositis. Incidences of up to 100% occur with some forms of chemotherapy or radiation therapy. Clinically significant mucositis develops with a range of standard chemotherapy drugs that are used, either alone or in combination, to treat various cancers including those of the colon, breast, prostate, head, neck and haemopoetic system. Examples of drugs that frequently cause mucositis include, but are not limited to, alkylating agents such as mechlorethamine, melphalan and busulphan, antimetabolites including cytarabine, floxuridine, 5-fluorouracil, mercaptopurine, methotrexate and thioguanine, cytotoxic drugs such as bleomycin, actinomycin-D, daunorubicin, cisplatin, etoposide, mitomycin, vinblastine and vincristine, and other chemotherapy drugs such as hyroxyurea and procarbazine (Sonis 1993). Direct exposure of the alimentary tract to high-dose radiotherapy or radiation, as occurs for example with total body irradiation, treatment of head and neck tumors or radiotherapy of abdominal tumors, will also cause a high incidence of mucositis.

One problem that is typically associated with mucositis is excessive weight loss. The damage inflicted upon the oral mucosa typically makes it painful for the patient to eat. This in turn leads to malnutrition, weight loss, and increased susceptibility to infections.

Enhanced susceptibility to infections is especially problematic in patients with stomatitis. Since the mouth is normally rich in microorganisms, the loss of mucosal integrity increases the risk of local and systemic bacterial infection, especially in patients with compromised immune systems.

Despite the widespread recognition that oral mucositis is a serious problem, no effective treatment exists today. Care is typically palliative. Analgesics such as morphine are given to control pain. Total parenteral nutrition can be used to provide nutrition. Antibiotics are used to control any infections arising from loss of mucosal integrity.

In addition to a lack of effective treatments for mucositis, physicians are unsure of the exact mechanism by which the ulcerations occur in mucositis. As described by Sonis et al, (Oral Oncology 34 (1998) 39-43) the initial exposure to chemotherapy causes a release of cytokines from the epithelial tissue. Subsequently, mitosis is disturbed in the epithelia. Finally, there are alterations in the bacterial flora of the oral cavity. It is unknown which of these occurrences, if any, is responsible for the mucosal damage. Despite the fact that significant quantities of inflammatory mediators are released by the epithelium, conventional anti-inflammatory agents have been unsuccessful in human efficacy studies of the disease. Thus, a significant need exists for alleviating oral mucositis.

### SUMMARY OF THE INVENTION

The present invention is directed to pharmaceutical compositions, including oral mouth rinse (mouthwash) or lozenge embodiments, comprising a compound corresponding to the formula A-B-X, or a pharmaceutically acceptable salt thereof, wherein "A "is a nucleoside structure selected from adenosine, guanosine, and uridine; "B" is a diphosphate linkage attached to the 5' carbon of the nucleoside ribose moiety; and "X" is attached to the disphosphate B as a moiety selected from hydrogen, furanose, or pyranose. The present invention is also directed to nutritional compositions or liquids having one or more nutrients in combination with the nucleoside derivatives described herein.

The present invention is also directed to a method of alleviating mucositis, said method comprising administering to a patient in need thereof a therapeutically effective amount of the nucleoside derivatives described herein. The present invention is also directed to the application of such methods using the pharmaceutical compositions of the present invention, including the mouth rinse and lozenge embodiments hereof, as well as the use of the liquid nutritional compositions of the present invention.

It has been found that the compositions of the present invention can be used to effectively alleviate mucositis in susceptible individuals. These compositions, when used in accordance with the methods of the present invention, can be administered to susceptible individuals prior to, during, or after treatments or events commonly associated with the development of mucositis such as certain chemotherapies, radiation therapies, or any other mucositis-inducing circumstance, to alleviate mucositis.

### BRIEF DISCRIPTIONS OF DRAWINGS

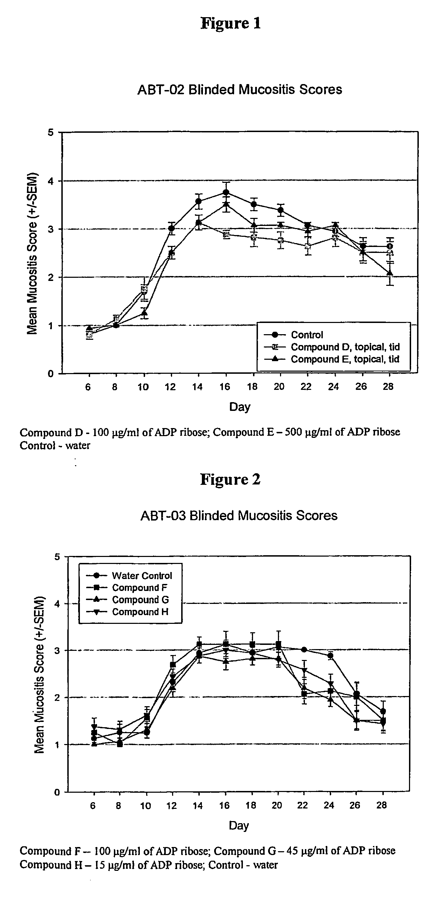

Figure 1 is a graph representing data from an animal study showing the effectiveness of a nucleoside derivative (ADP-ribose) in alleviating mucositis. The graph illustrates the average oral mucositis scores for three animal study groups defined by mucositis treatments comprising water (control), ADP-ribose at 100 ug/ml in water (Compound D), and ADP-ribose at 500 µg/ml (Compound E). The animal study is described in greater detail hereinafter.
Figure 2 is a graph representing data from a second animal study showing the effectiveness a nucleoside derivative (ADP-ribose) in alleviating mucositis. The graph illustrates the average oral mucositis scores for three animal study groups defined by mucositis treatments comprising water (control), ADP-ribose at 100 µg/ml (Compound F), ADP-ribose at 45 µg /ml in water (Compound G), and ADP-ribose at 15 µg/ml in water (Compound H). The animal study is described in greater detail hereinafter.

### DETAILED DESCRIPTION OF THE INVENTION

The compositions and methods of the present invention are all directed to certain nucleoside derivatives and their use in alleviating mucositis in susceptible individuals. These and other essential elements or limitations of the compositions and methods of the present invention are described in detail hereinafter.

The term "mucositis" as used herein, unless otherwise specified, refers to pain, redness, inflammation, ulceration, or combinations thereof, affecting the gastrointestinal tract from the mouth to anus, which results from disease or is secondary to therapeutic treatments such as certain chemotherapies, ionizing radiation, or combinations thereof, or is secondary to any mucositis-inducing circumstance or event. Non-limiting examples of specific types of mucositis include oral mucositis, esophagitis, enterititis, colitis, and combinations thereof.

The terms "oral mucositis" and "stomatitis" are used interchangeably herein and refer to mucositis affecting any surface of the oral pharyngeal and /or laryngeal epithelial surface, unless otherwise specified.

The term "esophagitis" as used herein, unless otherwise specified, refers to mucositis affecting the esophagus.

The term "alleviating" as use herein, unless otherwise specified, refers to preventing the occurrence of mucositis, decreasing the surface area of tissues that are affected by mucositis, reducing the intensity of mucositis, and/or enhancing or accelerating the rate at which these tissues heal and return to a normal or more normal state.

The term "pharmaceutical composition" as used herein, unless otherwise specified, refers to compositions suitable for use or prescribed treatment in alleviating mucositis in susceptible individuals.

The term "pharmaceutically acceptable salt" refers to those salts that are, within the scope of sound medical judgment, suitable for use in contact with the human tissue without undue toxicity, irritation, allergic response and the like and are commensurate with a reasonable benefit/risk ratio.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, 5, 6, from 1 to 9, from 3.6 to 4.6, from 3.5 to 9.9, and so forth.

All references to singular characteristics or limitations of the present invention shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

All percentages, parts and ratios as used herein are by weight of the total composition, unless otherwise specified. All such weights as they pertain to listed ingredients are based on the active level and, therefore, do not include solvents or by-products that may be included in commercially available materials, unless otherwise specified.

The compositions and methods of the present invention can comprise, consist of, or consist essentially of the essential elements and limitations of the invention described herein, as well as any additional or optional ingredients, components, or limitations described herein or otherwise useful in compositions and methods of the general type as described herein.

### NUCLEOSIDE DERIVATIVES

The compositions and methods of the present invention are directed to the formulation or use of selected nucleoside derivatives that conform to the formula A-B-X and are described in detail hereinafter. These selected nucleoside derivatives have been found to alleviate mucositis in susceptible individuals.

The selected nucleoside derivatives for use in the compositions and methods of the present invention conform to the general formula A-B-X , wherein "A" is a nucleoside structure selected from -adenosine, -guanosine, and -uridine; "B" is a diphosphate linkage attached to the 5'-carbon of the nucleoside ribose ring "A"; and; "X" is attached to B as a moiety selected from hydrogen, a furanose ring, or a pyranose ring. The selected nucleoside derivatives suitable for use herein also include all pharmaceutically acceptable salts thereof.

The ribose moiety of the nucleoside component "A", can be linked to the adenine, guanine or uracil base via an alpha or beta linkage at the anomeric carbon of the ribose moiety. The invention includes both embodiments (i.e. ribose linked to the base via either an alpha or a beta linkage).

The selected nucleoside derivative for use in the compositions and methods of the present invention preferably comprises adenosine for component "A" and ribose for component "X". This preferred compound is known as adenosine-diphosphate-ribose (also referred to herein as 5'-adenosine- diphosphate ribose or ADP-ribose). The ribose component of ADP-ribose signified by X, in the general formula, is covalently linked to the diphosphate linkage at carbon number 5 of the ribose group. As such, the "X" ribose contains an un-derivatized anomeric carbon that exists in aqueous solutions as an equilibrium of the alpha configuration five membered ring, the beta configuration five member ring, and as the open configuration, all of which are suitable for use herein.

The selected nucleoside derivative includes those compounds in which the "X" moiety is a pyranose structure such as glucose, galactose or mannose. While the linkage of "X" to the diphosphate bridge can be either an alpha or beta linkage at the anomeric carbon, the biologically active forms are typically in the alpha linkage, although both forms can be used in the compositions and methods herein.

Non-limiting examples of nucleoside derivatives suitable for use in the compositions and methods of the present invention include those compounds represented by the following chemical structures, wherein "X" is either hydrogen (Compound I), a pyranose moiety (Compound II), or furanose moiety (Compound III).

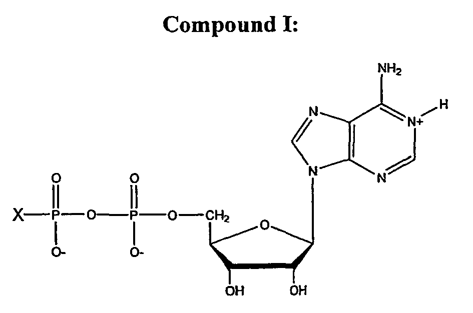

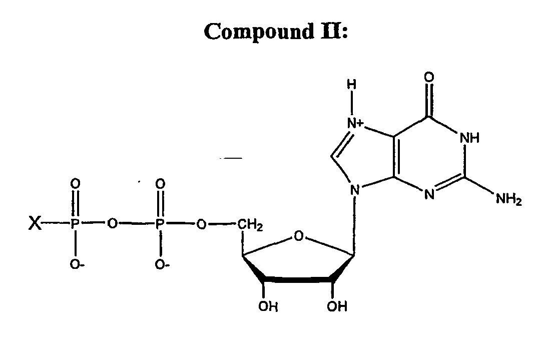

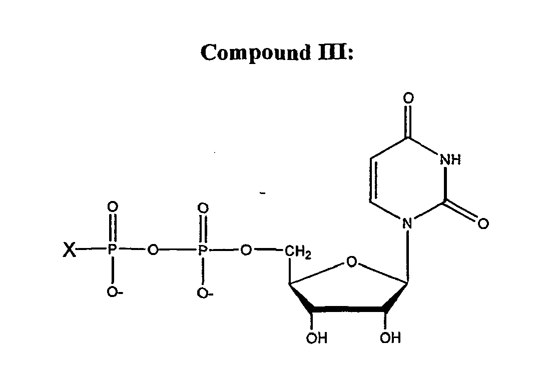

In the above-illustrated structures, the "X" moiety is either a hydrogen atom or a cyclic monosaccharide. When X is hydrogen, then the compounds are selected from the group consisting of 5' adenosine disphosphate, 5' guanosine diphosphate, and 5" uridine disphosphate (and the pharmaceutically acceptable salts thereof). These compounds are well known to those skilled in the art. They can be purchased from numerous commercial sources, including Sigma Chemical Corporation, St. Louis, Missouri, USA.

In the A-B-X structure described herein, when "X" is a monosaccharide, it will be either a pyranose or furanose moiety. As is readily apparent to those skilled in the art, these monosaccharides contain multiple asymmetric carbon atoms and therefore exist in the D-form or the L-form. As used herein, any reference to a pyranose other than fucose, or any furanose, refers to the D-configuration (i.e. the isomer present in nature). Fucose in both D- and L-configuration are suitable for use herein. As is also readily apparent to one skilled in the art, a cyclic monosaccharide contains an anomeric carbon atom and thus even the D-form can exist in one of two alternative forms, an alpha form (α) and a beta form (β). The pyranose and furanose moieties of the above-illustrated Compound I can therefore exist in either the beta form or the alpha form, either of which are useful in the compositions and methods of the present invention in alleviating mucositis.

In the A-B-X structure described herein, X will be D-ribose when X is a furanose structure. The "X" moiety can also be a pyranose structure such as glucose, galactose, mannose, or N-acetylglucosamine. Further, when the X is ribose, then A must be adenosine. When X is fucose, then A must be guanidine. When X is galactose, then A must be uridine. When X is mannose, then A must be adenosine or guanosine. When X is N-acetylglucosamine, then A must be uridine.

Non-limiting examples of suitable nucleoside derivatives for use in the compositions and methods of the present invention include: 5'-adenosine-diphosphate ribose; 5'-adenosine-diphosphate mannose; 5'-adenosine-diphosphate glucose; 5'-adenosine-diphosphate; 5'-guanosine-diphosphate mannose; 5'-guanosine-diphosphate-glucose; 5'-guanosine-diphosphate-fucose; 5'-uridine-diphosphate glucose; 5'-guanosine-diphosphate galactose; and 5'-guanosine-diphosphate N-acetylglucosamine.

All of the nucleoside derivatives as described herein are known in the various chemical arts, many of which are commercially available (e.g., 5-adenosine-diphosphate ribose can be purchased from Aldrich of St. Louis, Missouri, USA) while others can be prepared by techniques well known in the chemical arts, some methods of which are described in Gasmi et al, Cloning, expression, and characterization of YSAIH, a human adenosine 5'-diphospho sugar pyrophosphate possessing a MuT Motif, Biochem. J (1999), 344, 331-377.

The nucleoside derivatives as described herein include any known or otherwise effective pharmaceutically acceptable salt thereof, non-limiting examples of which include acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate, undecanoate, or combinations thereof.

The above-described pharmaceutically acceptable salts can also include those derivatives in which basic nitrogen-containing groups are quartemized with materials such as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and many others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of the nucleoside derivatives by reacting a carboxylic acid-containing moiety with a suitable base such as the hydroxide, carbonate or bicarbonate of a pharmaceutically acceptable metal action or with ammonia or an organic primary, secondary or tertiary amine. Non-limiting examples of pharmaceutically acceptable salts include those based on alkali metals or alkaline earth metals such as lithium, sodium, potassium, calcium, magnesium and aluminum salts and the like and nontoxic quaternary ammonia and amine captions including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine, and the like.

The nucleoside derivatives for use in the compositions and methods of the present invention are preferably used in an amount effective to alleviate mucositis as described herein, which in most instances will include a total daily dose of at least about 0.1 µg/kg/day, more typically from about 0.1 mg/kg/day to about 40 mg/kg/day, even more typically from about 1 mg/kg/day to about 20 mg/kg/day, although it is understood that the dose can vary considerably depending upon factors such as the dosage form selected, the targeted area of affected mucosa, and so forth. For most individuals, however, the total daily dose of the nucleoside derivative will be at least about 10 µg, more typically from about 10 µg to about 2000 mg, even more typically from about 2 mg to about 300 mg, per day. Any such dosage, however, should be evaluated in light of a reasonable risk/benefit ratio as is applicable with any medical treatment. It will be understood, however, that the total daily dosage of the nucleoside derivatives of the present invention will be decided by the attending physician within the scope of sound medical judgment. The specific dose for any patient will depend upon a variety of factors including the severity of the mucositis; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts.

The nucleoside derivative concentrations in the compositions of the present invention vary depending upon factors such as the particular product form selected, other selected ingredients in the composition, and so forth. For most oral liquid product forms, however, the concentrations will typically range from at least about 10 µg/ml, preferably from about 10µg/ml to about 1000µg/ml, more preferably from about 30 µg/ml to about 600 µg/ml, even more preferably from about 30 µg/ml to about 300 µg/ml.

If desired, the effective daily dose may be divided into multiple doses for purposes of administration. Consequently, single dose compositions may contain such amounts or submultiples thereof to make up the daily dose.

The compounds of the present invention are administered to a patient in need of such therapy (i.e. a patient at risk of, or suffering from mucositis). The route of administration may be by any appropriate method known to those skilled in the art. Typically however, a route will be used that will place the nucleoside derivatives directly in contact with the patients epithelial tissues, especially those of the mouth and esophagus. Such preferred routes include oral, nasal, and inhalation.

### PRODUCT FORMS

The compositions of the present invention, for use in the methods of the present invention, can be prepared in any known or otherwise effective dosage or product form suitable for use in providing topical or systemic delivery of the nucleoside derivatives to the affected mucosa, which would include both pharmaceutical dosage forms as well as nutritional product forms suitable for use in the methods described herein.

The compositions are preferably administered as oral dosage forms or products that rapidly coat or come in contact with the oral and/or esophageal mucosa, to thus provide more effective contact with the affected mucosal tissue. Preferred dosage or product forms in this respect include mouthwashes which the individual may swish and swallow or swish and spit out. Also preferred are oral lozenges.

The compositions and methods of the present invention are useful in any pharmaceutical or nutritional liquid product form that can directly or indirectly affect those areas of mucosa which have become or will likely become irritated due to chemical, viral, radiation, or other forms of irritation. For example, the compositions of the present invention can be formulated in product forms to treat individuals suffering from the mucosal irritation associated with diarrhea or microbial infections such as influenza, rhino viruses, or other microbial infections that can irritate the mucosa.

The pharmaceutical and liquid nutritional product forms are described hereinafter in greater detail.

### LIQUID NUTRITIONALS

The compositions of the present invention include liquid nutritional embodiments for oral or enteral administration that comprise one or more nutrients such as fats, carbohydrates, proteins, vitamins, and minerals. Oral liquid nutritionals are preferred.

These nutritional liquids are preferably formulated with sufficient viscosity, flow, or other physical or chemical characteristics to provide a more effective and soothing coating of the affected mucosa while drinking or administering the nutritional liquid. These nutritional embodiments also preferably represent a balanced nutritional source suitable for meeting the sole, primary, or supplemental nutrition needs of the individual.

Non-limiting examples of suitable nutritional liquids within which the nucleoside derivatives can be formulated, and thus form selected nutritional liquid embodiments of the present invention, are described in U.S. Patent 5,700,782 (Hwang et al.); U.S. Patent 5,869,118 (Morris et al.); and U.S. Patent 5,223,285 (DeMichele et al.).

Many different sources and types of carbohydrates, lipids, proteins, minerals and vitamins are known and can be used in the nutritional liquid embodiments of the present invention, provided that such nutrients are compatible with the added ingredients in the selected formulation, are safe and effective for their intended use, and do not otherwise unduly impair product performance.

Proteins suitable for use herein can be hydrolyzed, partially hydrolyzed or non-hydrolyzed, and can be derived from any known or otherwise suitable source such as milk (e.g., casein, whey), animal (e.g., meat, fish), cereal (e.g., rice, corn), vegetable (e.g., soy), or combinations thereof.

Fats or lipids suitable for use in the nutritional liquids include, but are not limited to, coconut oil, soy oil, corn oil, olive oil, safflower oil, high oleic safflower oil, MCT oil (medium chain triglycerides), sunflower oil, high oleic sunflower oil, structured triglycerides, palm and palm kernel oils, palm olein, canola oil, marine oils, cottonseed oils, and combinations thereof

Carbohydrates suitable for use in the nutritional liquids may be simple or complex, lactose-containing or lactose-free, or combinations thereof. Non-limiting examples of suitable carbohydrates include hydrolyzed corn starch, maltodextrin, glucose polymers, sucrose, corn syrup, corn syrup solids, rice-derived carbohydrate, glucose, fructose, lactose, high fructose corn syrup and indigestible oligosaccharides such as fructooligosaccharides (FOS), and combinations thereof.

The nutritional liquids may further comprise any of a variety of vitamins, non-limiting examples of which include vitamin A, vitamin D, vitamin E, vitamin K, thiamine, riboflavin, pyridoxine, vitamin B₁₂, niacin, folic acid, pantothenic acid, biotin, vitamin C, choline, inositol, salts and derivatives thereof, and combinations thereof.

The nutritional liquids may further comprise any of a variety of electrolytes known or otherwise suitable for us in patients at risk of or suffering from mucositis, non-limiting examples of which include calcium, phosphorus, magnesium iron, selenium, manganese, copper, iodine, sodium, potassium, chloride, and combinations thereof.

The liquid nutritional embodiments preferably comprise a combination of carbohydrate, protein and lipid, wherein each nutrient is formulated within the ranges described in the following table:

| Nutrient | Range | gm/100 kcal | gm/liter |
|---|---|---|---|
| Carbohydrate | Preferred | 8-16 | 54-108 |
| | More preferred | 9-13 | 61-88 |
| Lipid | Preferred | 3-8 | 20-54 |
| | More preferred | 4-6.6 | 27-45 |
| Protein | Preferred | 1-3.5 | 7-24 |
| | More preferred | 1.5-3.4 | 10-23 |

The nutritional liquids preferably include per 100 kcal one or more of the following: vitamin A (from about 250 to about 750 IU), vitamin D (from about 40 to about 100 IU), vitamin K (greater than about 4 µm), vitamin E (at least about 0.3 IU), vitamin C (at least about 8 mg), thiamine (at least about 8 µg), vitamin B₁₂ (at least about 0.15 µg), niacin (at least about 250 µg), folic acid (at least about 4 µg), pantothenic acid (at least about 300 µg), biotin (at least about 1.5 µg), choline (at least about 7 mg), and inositol (at least about 4 mg).

The nutritional liquids also preferably include per 100 kcal one or more of the following: calcium (at least about 50 mg), phosphorus (at least about 25 mg), magnesium (at least about 6 mg), iron (at least about 0.15 mg), iodine (at least about 5 µg), zinc (at least about 0.5 mg), copper (at least about 60 µg), selenium (at least about 50 µg), manganese (at least about 5 µg), sodium (from about 20 to about 60 mg), potassium (from about 80 to about 200 mg), and chloride (from about 55 to about 150 mg).

The nutritional liquids may further comprise other optional components that may modify the physical, chemical, aesthetic or processing characteristics of the liquid composition or serve as pharmaceutical or additional nutritional components when used in the targeted population. Many such optional ingredients are known for use in nutritional products and may also be used in the nutritional liquid embodiments of the present invention, provided that such optional materials are compatible with the essential materials described herein, are safe and effective for their intended use, and do not otherwise unduly impair product performance. Non-limiting examples of such optional ingredients include preservatives, additional anti-oxidants, emulsifying agents, buffers, colorants, flavors, thickening agents, fiber, stabilizers, and so forth.

### PHARMACEUTICAL COMPOSITIONS

The pharmaceutical compositions of the present invention can be prepared by any known or otherwise effective method for formulating or manufacturing the selected product form. For example, the nucleoside derivatives can be formulated along with common excipients, diluents, or carriers, and formed into oral tablets, capsules, sprays, mouth washes, lozenges, treated substrates (e.g., oral or topical swabs, pads, or disposable, non-digestible substrate treated with the compositions of the present invention); oral liquids (e.g., suspensions, solutions, emulsions), powders, or any other suitable dosage form.

Non-limiting examples of suitable excipients, diluents, and carriers include: fillers and extenders such as starch, sugars, mannitol, and silicic derivatives; binding agents such as carboxymethyl cellulose and other cellulose derivatives, alginates, gelatin, and polyvinyl pyrolidone; moisturizing agents such as glycerol; disintegrating agents such as calcium carbonate and sodium bicarbonate; agents for retarding dissolution such as paraffin; resorption accelerators such as quaternary ammonium compounds; surface active agents such as acetyl alcohol, glycerol monostearate; adsorptive carriers such as kaolin and bentonite; carriers such as propylene glycol and ethyl alcohol, and lubricants such as talc, calcium and magnesium stearate, and solid polyethyl glycols.

The nucleoside derivatives described herein can also be formulated as elixirs or solutions for convenient oral administration or as solutions appropriate for parenteral administration, for instance by intramuscular, subcutaneous or intravenous routes. Additionally, the nucleoside derivatives are also well suited for formulation as a sustained or prolonged release dosage forms, including dosage forms that release active ingredient only or preferably in a particular part of the intestinal tract, preferably over an extended or prolonged period of time to further enhance effectiveness. The coatings, envelopes, and protective matrices in such dosage forms may be made, for example, from polymeric substances or waxes well known in the pharmaceutical arts.

The compositions of the present invention include pharmaceutical dosage forms such as lozenges, troches or pastilles. These are typically discoid-shaped solids containing the active ingredient in a suitably flavored base. The base may be a hard sugar candy, glycerinated gelatin, or the combination of sugar with sufficient mucilage to give it form. Troches are placed in the mouth where they slowly dissolve, liberating the active ingredient for direct contact with the affected mucosa.

The troche embodiments of the present invention can be prepared, for example, by adding water slowly to a mixture of the powdered active, powdered sugar, and a gum until a pliable mass is formed. A 7% acacia powder can be used to provide sufficient adhesiveness to the mass. The mass is rolled out and the troche pieces cut from the flattened mass, or the mass can be rolled into a cylinder and divided. Each cut or divided piece is shaped and allowed to dry, to thus form the troche dosage form.

If the active ingredient is heat stable, or can be rendered heat stable by the use of appropriate processing precautions, it may be prepared in the form of a hard candy base. For example, sugar-containing syrup can be concentrated to the point where it becomes a pliable mass. The active ingredient is then added to the mass, which is then kneaded while warm to form a homogeneous mass. The homogeneous mass is gradually worked into a pipe form having the diameter desired for the candy piece. Lozenges can be cut or sectioned off from the pipe and allowed to cool.

If the active ingredient is heat labile, it may be made into a lozenge preparation by compression. For example, the granulation step in the preparation is performed in a manner similar to that used for any compressed tablet. The lozenge is made using heavy compression equipment to give a tablet that is harder than usual as it is desirable for the dosage form to dissolve or disintegrate slowly in the mouth. Ingredients are preferably selected to promote slow-dissolving characteristics.

For nasal administration, the nucleoside derivatives may be dissolved in a physiologically acceptable pharmaceutical carrier and administered as a solution or spray. Illustrative of suitable pharmaceutical carriers are water, saline, and aqueous alcoholic solutions. The pharmaceutical carrier may also contain preservatives, buffers, or other material suitable for such a dosage form.

For inhalation therapy, the nucleoside derivates can be incorporated into an aqueous alcoholic solution containing a fluorinated hydrocarbon propellant and packaged into a suitable administration device as known in the art.

### EXAMPLES

The exemplified pharmaceutical and nutritional compositions of the present invention, all of which are used in accordance with the methods of the present invention, may be prepared by any known or otherwise effective technique, suitable for making and formulating pharmaceutical dosage forms or nutritional formulas. Many such methods are described in the pharmaceutical and nutritional arts or are otherwise well known to those skilled in their respective formulation arts.

The following examples further describe and demonstrate specific embodiments within the scope of the present invention. The examples are given solely for the purpose of illustration and are not to be construed as limitations of the present invention, as many variations thereof are possible without departing from the spirit and scope of the invention. All exemplified amounts are weight percentages based upon the total weight of the composition, unless otherwise specified.

Unless otherwise specified, the active ingredient in each example is a nucleoside derivative as described herein. Each example is formulated and repeated with each of the following active ingredients: 5'-adenosine -diphosphate ribose; 5'-adenosine-diphosphate mannose; 5'-adenosine-diphosphate glucose; 5'-adenosine-diphosphate; 5'-guanosine-diphosphate mannose; 5'-guanosine-diphosphate-glucose; 5'-guanosine-diphosphate-fucose; 5'-uridine-diphosphate glucose; 5'-guanosine-diphosphate galactose; and 5'-guanosine-diphosphate N-acetylglucosamine.

### Example 1

The following exemplified dosage forms illustrate some of the possible pharmaceutical formulation embodiments of the present invention. It can be used in accordance with the methods of the present invention to alleviate mucositis in susceptible or otherwise affected individuals.

### 1.1 Gelatin Capsules

Hard gelatin capsules are prepared which contain, per capsule, from about 0.01 mg to about 2000 mg of active ingredient, from zero to about 650mg of Starch NF, from zero to about 650mg of starch flowable powder; and from zero to 15mg silicone fluid 350 centistokes; wherein the ingredients are blended together, passed through a No. 45 mesh U.S. sieve, and filled into hard gelatin capsules. The capsules are then administered orally to patients to alleviate mucositis in accordance with the methods of the present invention. Specific exemplified capsule dosage forms are described below:

| FORMULATION | mg/capsule |
|---|---|
| Formulation 1.1.1 | |
| Active ingredient | 1.0 |
| Starch, NF | 112 |
| Starch flowable powder | 225.3 |
| Silicone fluid 350 cs | 1.7 |
| | |

| Formulation 1.1.2 | |
|---|---|
| Active ingredient | 5 |
| Starch, NF | 108 |
| Starch flowable powder | 225.3 |
| Silicone fluid 350 cs | 1.7 |
| | |

| Formulation 1.1.3 | |
|---|---|
| Active ingredient | 10 |
| Starch, NF | 103 |
| Starch flowable powder | 225.3 |
| Silicone fluid 350 cs | 1.7 |
| | |

| Formulation 1.1.4 | |
|---|---|
| Active ingredient | 50 |
| Starch, NF | 150 |
| Starch flowable powder | 397 |
| Silicone fluid 350 cs 3.0 | |

### 1.2 Tablets

Tablet embodiments of the present invention are prepared which contain, per tablet, from about 0.01 to about 1000 mg of active ingredient, from zero to about 650mg of microcrystalline cellulose; from zero to about 650mg of fumed silicon dioxide; and from zero to 15mg stearic acid; wherein the various ingredients are blended together and compressed into tablets. The tablets are then administered orally to patients to alleviate mucositis in accordance with the methods of the present invention. Other specific tablet dosage forms are described below:

| Formulation 1.2.1 | mg/capsule |
|---|---|
| Active ingredient | 0.01-1000 |
| Starch | 45 |
| Cellulose, microcrystalline | 35 |
| Polyvinylpyrrolidone | 4 |
| (as 10% solution in water) | |
| Sodium carboxymethyl cellulose | 4.5 |
| Magnesium stearate | 0.5 |
| Talc | 1 |

Formulation 1.2.1 is prepared by passing the active ingredient, starch, and cellulose through a No. 45 mesh U.S. sieve and thoroughly mixing the particles together. The polyvinylpyrrolidone solution is mixed with the resultant powders which are then passed through a No. 14 mesh U.S. sieve. The resulting granules are dried at 50-60° C and then passed through a No. 18 mesh U.S. sieve. The sodium carboxymethyl starch, magnesium stearate, and talc, previously passed through a No. 60 U.S. sieve, are then added to and thoroughly mixed with the processed granules. The resulting combination is then compressed into tablets.

### 1.3 Liquid Suspension

Liquid suspension embodiments of the present invention are prepared by conventional formulation techniques, each containing from about 0.01 mg to about 2000 mg of active ingredient per 5 ml of suspension. The suspensions are then administered orally as a mouthwash to patients to alleviate mucositis in accordance with the methods of the present invention. Specific suspension embodiments are described below

| Formulation 1.3.1 | (mg/5 ml) |
|---|---|
| Active ingredient | 0.1-2000 |
| Sodium carboxymethyl cellulose | 50 |
| Syrup | 1.25 |
| Benzoic acid solution | 0.10 ml |
| Flavor | q.v. |
| Color | q.v. |
| Purified water to | 5 ml |

Formulation 1.3.1 is prepared by passing the active ingredient is through a No. 45 mesh U.S. sieve and mixing it with sodium carboxymethyl cellulose and syrup to form a smooth paste. The benzoic acid solution, flavor, and color are diluted with some of the water and added, with stirring. Sufficient water is then added to produce the required suspension volume.

| Formulation 1.3.2 | mg/5ml |
|---|---|
| Active ingredient | 0.01-2000 mg |
| Chlorobutanol | 0.5 g |
| Sodium Chloride | 0.5 g |
| Water | QS 100 ml |

Formulation 1.3.2 is prepared by passing the active ingredient through a No. 45 mesh U.S. sieve and mixing it with sodium chloride, chlorobutanol, and water with stirring.

### 1.4 Sublingual or Buccal Tablets

Sublingual or buccal tablet embodiments of the present invention are prepared to contain the following ingredients per tablet:

| | |
|---|---|
| Active ingredient | 0.1-1000 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |

The glycerol, water, sodium citrate, polyvinyl alcohol, and polyvinylpyrrolidone are mixed together by continuous stirring and at a temperature of about 90° C. When the polymers have gone into solution, the solution is cooled to about 50-55° C. and the active ingredient is slowly admixed. The homogenous mixture is poured into forms made of an inert material to produce an active ingredient-containing diffusion matrix having a thickness of about 2-4mm. This diffusion matrix is then cut to form individual tablets having the appropriate size. The tablets are then administered orally (e.g. sublingual or buccal) to patients to alleviate mucositis in accordance with the methods of the present invention.

### Example 2

This example illustrates a nutritional liquid embodiment of the present invention, including a method of using and making the formula. The ingredients for this exemplified embodiment are listed in the following table:

| **INGREDIENT** | **AMOUNT** |
|---|---|
| Water | 31,605.21 kg |
| Gum Arabic | 437.84 kg |
| Ultratrace/Tracemineral Premix | 14.50 kg |
| Potassium citrate | 50.00 kg |
| Sodium citrate | 95.00 kg |
| Potassium iodide | 9.00 gm |
| Potassium chloride | 91.00 kg |
| Corn syrup solids | 5630.96 kg |
| Maltodextrin | 1407.52 kg |
| Magnesium phosphate dibasic | 131.00 kg |
| Calcium phosphate tribasic | 47.50 kg |
| Calcium carbonate | 122.50 kg |
| Sugar (sucrose) | 852.77 kg |
| Fructooligosaccharide | 509.96 kg |
| Medium chain triglycerides (fractionated coconut oil) | 172.69 kg |
| Canola oil | 99.13 kg |
| Sol oil | 58.63 kg |
| 57% Vitamin palmitate | 250.00 gm |
| 2.5% Vitamin D | 35.00 gm |
| D-alpha tocopherol acetate (R,R,R) | 10.65 kg |
| Phylloquine | 6.50 gm |
| 30% Beta carotene | 824.00 gm |
| Soy lecithin | 42.64 kg |
| Sodium caseinate | 1427.04 kg |
| Partially hydrolyzed sodium caseinate | 1427.04 kg |
| Soy polysaccharide | 85.28 kg |
| 75% Whey protein concentrate | 184.46 kg |
| Refined deodorized sardine oil | 692.87 kg |
| Ascorbic acid | 37.08 kg |
| 45% Potasssium hydroxide | 25.96 kg |
| Taurine | 12.00 kg |
| Water soluble vitamin premix | 4.50 kg |
| Folic acid | 43.50 gm |
| Choline chloride | 25.00 kg |
| L-Carnitine | 7.00 kg |
| Flavorant | 49.9 kg |
| ADP-Ribose | 15 gm |

The exemplified formula can be manufactured by any known or otherwise effective method for preparing liquid nutritional formulas, non-limiting examples of which are described in US Patent 6,326,355 (Abbruzzese et al.), which descriptions are incorporated herein by reference. These methods are typically modified by the addition of ADP-ribose to an aqueous carbohydrate slurry formed during an initial processing step, so that the finished product most typically contains from about 10 to about 1000 µg of ADP-ribose per ml of the final formula product. The above-formula contains about 300 µg/ml of ADP-ribose.

The above formula is also modified using other nutritional base formulas, each with 45-55 µg/ml of ADP-ribose, including commercially available nutritional liquids: Ensure ®, Puhnocare ®, Prosure ®, and Osmolite ® (all available from Ross Products Division of Abbot Labs, Columbus, Ohio USA). The above formula is also substituted with other nutritional base materials Boost®, Boost Breeze®, Boost® High Protein, Boost® High Protein Powder, Boost Plus®, Boost® Pudding, Boost® with Fiber, ChoiceDM® Beverage, ChoiceDM® Beverage, Subdue®, Subdue Plus®, Criticare HN®, Isocal®, Isocal® HN, Isocal® HN Plus, Kindercal®, Kindercal® TF, Lipisorb® Liquid, Magnacal® Renal, Microlipid®, TraumaCal®, Ultracal®, and Ultracal® HN Plus (all available from Mead Johnson & Company, Evansville, Indiana USA).

Each of the many exemplified nutritional liquids is used in accordance with the methods of the present invention to alleviate mucositis as described herein. These products can be swished around the inside of the mouth before swallowing to enhance the local affect of the applied formula on the oral mucosa. The swallowed formula is then made available to the mucosal areas below the oral cavity, and when absorbed, systemically available to further alleviate mucositis.

The nutritional liquid embodiments of the present invention, including the many exemplified formulas described hereinbefore, are unique in that they provide a combination of much needed benefits. First, they contact and sooth the oral mucosa and other affected areas of the GI tract, and thus often provide some temporary relief from the discomfort of an irritated or damaged mucosa. Second, it provides topical delivery of ADP-ribose or other selected nucleoside derivatives to the affected areas during administration as well as systemic delivery after absorption. And third, it often provides much needed nutrition in those people suffering from the more developed stages of mucositis who find it difficult to eat due to the discomfort associated with mucositis.

The exemplified nutritional liquid formula is reformulated with greater and lesser amounts of ADP-ribose, resulting in formulas similar to the above formula except ADP-ribose concentrations are modified to 10, 50, 100, 250, and 500 µg/ml in the finished formula, each of which is used orally to provide nutrition and to alleviate mucositis in susceptible individuals.

### Example 3 - Animal Study

The objective of this study was to evaluate the effect of the nucleoside derivative, 5'-adenosine-diphosphate ribose, when administered topically, on the frequency, severity and duration of oral mucositis induced by acute radiation in an animal model.

The animals in the study (32 Golden Syrian hamsters, Charles River Lab., ages 5-6 weeks, approximately 90 g average body weight) were randomly and prospectively divided into three groups of eight animals each. The particular test material to be applied to the animal mucosa during the study essentially defined each group. The test materials were water (Group 1 control), ADP-ribose 100 µg/ml in water (Group 2) and ADP-ribose 500 µg/ml in water (Group 3)

Mucositis was induced in the animals with a standardized acute radiation protocol. A single dose of radiation (40 Gy/dose) was administered to each animal on day 0. Irradiation targeted the left buccal pouch mucosa at a rate of 121.5 cGy/minute. The left buccal pouch was everted, fixed and isolated using a lead shield. Prior to and following radiation on day 0, each animal was dosed with their respective test materials in the manner described below. Also, immediately prior to radiation, the buccal mucosa was everted and any excess test material solution was wiped away using a clean tissue or cotton swab.

The respective test materials were then applied topically TID to the irradiated mucosa of each animal on day 1 to day 20 of the study. To accomplish this, a tuberculin syringe without a needle, containing 0.25 ml of the test material, was inserted into the base of the left cheek pouch and the test material deposited accordingly. This was done TID (first between 8-9:00 am; second between 12:00 am and 1:00 pm; third between 4-6:00 pm). Any developing mucositis was evaluated and scored on alternate days from day 6-28. Each animal was also weighed and its survival recorded on day 1 to day28.

During the study, each animal was tested for mucositis scores, weight change and survival. For the evaluation of mucositis, the animals were anesthetized with inhalation anesthetics, and the left pouch everted. The developing mucositis was scored visually by comparison to a validated photographic scale, ranging from 0 for normal, to 5 for severe ulceration (clinical scoring). In descriptive terms, this scale is defmed in the following table:

| **Score:** | **Description:** |
|---|---|
| **0** | Pouch completely healthy. No erythema or vasodilation |
| **1** | Light to severe erythema and vasodilation. No erosion of mucosa |
| **2** | Severe erythema and vasodilation. Erosion of superficial aspects of mucosa leaving denuded areas. Decreased stippling of mucosa. |
| **3** | Formation of off-white ulcers in one or more places. Ulcers may have a yellow/gray due to pseudomembrane. Cumulative size of ulcers should equal about ¼ of the pouch. Severe erythema and vasodilation. |
| **4** | Cumulative seize of ulcers should equal about ½ of the pouch. Loss of pliability. Severe erythema and vasodilation. |
| **5** | Virtually all of pouch is ulcerated. Loss of pliability (pouch can only partially be extracted from mouth) |

A mucositis score of 1-2 represents a mild stage of the disease. A score of 3-5 indicates moderate to severe mucositis. Following visual scoring, a photograph was taken of each animal's mucosa using a standardized technique. At the conclusion of the experiment, all film was developed and the photographs randomly numbered. At least two independent trained observers graded the photographs in blinded fashion using the above-described scale (blinded scoring). Results of the mucositis scores are graphically illustrated in Figure 1.

Figure 1 shows the average oral mucositis scores for the three study groups. In the figure above, the control group was treated with water; Compound D group was treated with ADP-ribose at 100 ug/ml in water, and Compound E group was treated with ADP-ribose at 500 µg/ml. As shown in Figure 1, the application of ADP-ribose at both concentrations decreased the severity of oral mucositis in the animal model. It can also be noted that the application of ADP-ribose at 100 µg/ml had an effect superior to that of ADP-ribose at 500 µg/ml.

### Example 4 - Animal Study II

The animal study described in Example 2 was then repeated with an identical protocol but with modified ADP-ribose doses. The groups in this study were treated with water (control), ADP-ribose at 100 µg/ml in water (Compound F), ADP-ribose at 45µg/ml in water (Compound G), and ADP-ribose at 15 µg/ml in water (Compound H). The results of the second study are shown in Figure 3. The data shows that the ulcers associated with mucositis (i.e., a mucositis score≥ 3) healed faster in the ADP-ribose treatment groups relative to the control. Animals in the control group had mucositis scores ≥ 3 for approximately 10 days, whereas the ADP-ribose group had scores of approximately 3 for only 4 days. Moreover, ADP-ribose application at a 45 µg/ml dose provided maximum healing of oral mucositis that was statistically significant.

## Claims

1. The use of a formula comprising a nucleoside derivative that conforms to the general formula A-B-X or a pharmaceutically acceptable salt thereof, wherein, A is a nucleoside structure selected from the group consisting of adenosine, guanosine, and uridine; B is a diphosphate linkage attached to a 5'-carbon of a nucleoside ribose ring of the nucleoside structure A; and X is a substituent attached to the diphosphate linkage B and selected from the group consisting of hydrogen, a furanose ring, or a pyranose ring;
for manufacturing a medicament for use in alleviating mucositis by admimistering to an individual in need thereof a therapeutically effective amount of the nucleoside derivative.

2. A use according to claim 1 wherein A is adenosine.

3. A use according to claim 1 wherein X is a pyranose ring selected from the group consisting of glucose, galactose, mannose, N-acetylglucosmine, or fucose.

4. A use according to claim 1 wherein X is ribose.

5. A use according to claim 1 wherein the nucleoside derivative is 5'-adenosine-diphosphate ribose.

6. A use according to any one of the preceding claims wherein the nucleoside derivative is administered at a dose of from 0.1 µg/kg/day to 40 mg/kg/day, preferably from 10 µg/day to 2000 mg/day.

7. A use according to any one of the preceding claims wherein the nucleoside derivative is administered orally.

8. A use according to any one of the preceding claims wherein the nucleoside derivative is topically administered to the oral mucosa.

9. Use of a composition for manufacturing a medicament for treating mucositis, said composition comprising a therapeutically effective amount of a nucleoside derivative that conforms to the general formula A-B-X or a pharmaceutically acceptable salt thereof, wherein, A is a nucleoside structure selected from the group consisting of adenosine, guanosine, and uridine; B is a diphosphate linkage artached to a 5'-carbon of a nucleoside ribose ring of the nucleoside structure A; and X is a substituent attached to the diphosphare linkage B and selected from the group consisting of hydrogen, a furanose ring or a pyranose ring.

10. A use according to claim 9 wherein A is adenosine.

11. A use according to 9 wherein X is a pyranose ring selected from the group consisting of glucose, galactose, mannose, N-aretylglucosamine, or fucose.

12. A use according to 9 wherein X is ribose.

13. A use according to 9 wherein the nucleoside derivative is 5'-adenosine-diphosphate ribose.

14. A use according to any one of claims 9 to 13 wherein the composition is in the form of an oral lozenge.

15. A use according to any one of claims 9-13 wherein the composition is an oral composition, preferably a mouthwash.

16. A use according to claim 15 wherein the mouthwash comprises 5'-adenosine- diphosphate ribose at a concentration of at least 10 µg/ml, preferably from 10 µg/ml to 1000 µg/ml.

17. A use according to any one of claims 9-13 wherein the composition is an oral nutritional liquid further comprising protein, carbohydrate, and fat.

18. An oral composition comprising a therapeutically effective amount of 5'-adenosine-diphosphate ribose for use in the treatment of mucositis.

## Patentansprüche

1. Die Verwendung einer Formulierung umfassend ein Nukleosidderivat, das der allgemeinen Formel A-B-X entspricht, oder ein pharmazeutisch verträgliches Salz davon, worin A eine Nukleosidstruktur ist gewählt aus der Gruppe bestehend aus Adenosin, Guanosin und Uridin; B ist eine Diphosphatverbindung angeheftet an einen 5'-Kohlenstoff eines Nukleosidriboserings der Nukleosidstruktur A; und X ist ein Substituent angeheftet an die Diphosphatverbindung B und gewählt aus der Gruppe bestehend aus Wasserstoff, einem Furanosering oder einem Pyranosering; zur Herstellung eines Medikaments zur Verwendung in der Linderung von Mukositis durch Verabreichen einer therapeutisch wirksamen Menge des Nukleosidderivats an eine Person, die das benötigt.

2. Eine Verwendung gemäß Anspruch 1, worin A Adenosin ist.

3. Eine Verwendung gemäß Anspruch 1, worin X ein Pyranosering ist, gewählt aus der Gruppe bestehend aus Glucose, Galactose, Mannose, N-Acetylglucosamin oder Fucose.

4. Eine Verwendung gemäß Anspruch 1, worin X Ribose ist.

5. Eine Verwendung gemäß Anspruch 1, worin das Nukleosidderivat 5'-Adenosindiphosphat Ribose ist.

6. Eine Verwendung gemäß irgendeinem der vorhergehenden Ansprüche, worin das Nukleosidderivat in einer Dosis von 0,1 µg/kg/Tag bis 40 mg/kg/Tag, vorzugsweise von 10 µg/Tag bis 2000 mg/Tag, verabreicht wird.

7. Eine Verwendung gemäß irgendeinem der vorhergehenden Ansprüche, worin das Nukleosidderivat oral verabreicht wird.

8. Eine Verwendung gemäß irgendeinem der vorhergehenden Ansprüche, worin das Nukleosidderivat topisch an die Mundschleimhaut verabreicht wird.

9. Verwendung einer Zusammensetzung zur Herstellung eines Medikaments zur Behandlung von Mukositis, wobei die Zusammensetzung eine therapeutisch wirksame Menge eines Nukleosidderivats umfaßt, das der allgemeinen Formel A-B-X entspricht, oder ein pharmazeutisch verträgliches Salz davon, worin A eine Nukleosidstruktur ist, gewählt aus der Gruppe bestehend aus Adenosin, Guanosin und Uridin; B ist eine Diphosphatverbindung angeheftet an einen 5'-Kohlenstoff eines Nukleosidriboserings der Nukleosidstruktur A; und X ist ein Substituent angeheftet an die Diphosphatverbindung B und gewählt aus der Gruppe bestehend aus Wasserstoff, einem Furanosering oder einem Pyranosering.

10. Eine Verwendung gemäß Anspruch 9, worin A Adenosin ist.

11. Eine Verwendung gemäß 9, worin X ein Pyranosering gewählt aus der Gruppe bestehend aus Glucose, Galactose, Mannose, N-Acetylgluxosamin oder Fucose ist.

12. Eine Verwendung gemäß 9, worin X Ribose ist.

13. Eine Verwendung gemäß 9, worin das Nucleosidderivat 5'-Adenosindiphosphat Ribose ist.

14. Eine Verwendung gemäß irgendeinem der Ansprüche 9 bis 13, worin die Zusammensetzung in der Form einer oralen Pastille vorliegt.

15. Eine Verwendung gemäß irgendeinem der Ansprüche 9-13, worin die Zusammensetzung eine orale Zusammensetzung, vorzugsweise ein Mundwasser, ist.

16. Eine Verwendung gemäß Anspruch 15, worin das Mundwasser 5'-Adenosindiphosphatribose bei einer Konzentration von mindestens 10 µg/ml, vorzugsweise von 10 µg/ml bis 1000 µg/ml, umfaßt.

17. Eine Verwendung gemäß irgendeinem der Ansprüche 9-13, worin die Zusammensetzung eine orale Ernährungsflüssigkeit ist, die weiter Protein, Kohlenhydrat und Fett umfasst.

18. Eine orale Zusammensetzung umfassend eine therapeutisch wirksame Menge von 5'-Adenosindiphosphat Ribose für die Verwendung in der Behandlung von Mukositis.

## Revendications

1. Utilisation d'une formule comprenant un dérivé de nucléoside qui est conforme à la formule générale A-B-X ou d'un sel pharmaceutiquement acceptable de celui-ci, dans laquelle A est une structure nucléoside choisie parmi l'adénosine, la guanosine et l'uridine ; B est une liaison diphosphate fixée à un carbone 5' d'un noyau nucléoside ribose de la structure nucléoside A ; et X est un substituant fixé à la liaison diphosphate B et choisi parmi un atome d'hydrogène, un noyau furanose ou un noyau pyranose ;
pour la fabrication d'un médicament destiné à être utilisé pour soulager une inflammation d'une muqueuse par administration à un individu nécessitant celui-ci d'une quantité thérapeutiquement efficace du dérivé de nucléoside.

2. Utilisation selon la revendication 1, dans laquelle A est l'adénosine.

3. Utilisation selon la revendication 1, dans laquelle X est un noyau pyranose choisi parmi le glucose, le galactose, le mannose, la N-acétylglucosamine ou le fucose.

4. Utilisation selon la revendication 1, dans laquelle X est le ribose.

5. Utilisation selon la revendication 1, dans laquelle le dérivé de nucléoside est le 5'-adénosine-diphosphate ribose.

6. Utilisation selon l'une quelconque des revendications précédentes, dans laquelle le dérivé de nucléoside est administré à une dose de 0,1 µg/kg/jour à 40 mg/kg/jour, de préférence de 10 µg/jour à 2 000 mg/jour.

7. Utilisation selon l'une quelconque des revendications précédentes, dans laquelle le dérivé de nucléoside est administré par voie orale.

8. Utilisation selon l'une quelconque des revendications précédentes, dans laquelle le dérivé de nucléoside est administré par voie topique à la muqueuse orale.

9. Utilisation d'une composition pour la fabrication d'un médicament destiné au traitement d'une inflammation d'une muqueuse, ladite composition comprenant une quantité thérapeutiquement efficace d'un dérivé de nucléoside qui est conforme à la formule générale A-B-X ou d'un sel pharmaceutiquement acceptable de celui-ci, dans laquelle A est une structure nucléoside choisie parmi l'adénosine, la guanosine et l'uridine ; B est une liaison diphosphate fixée à un carbone 5' d'un noyau de nucléoside ribose de la structure nucléoside A ; et X est un substituant fixé à la liaison diphosphate B et choisi parmi un atome d'hydrogène, un noyau furanose ou un noyau pyranose.

10. Utilisation selon la revendication 9, dans laquelle A est l'adénosine.

11. Utilisation selon la revendication 9, dans laquelle X est un noyau pyranose choisi parmi le glucose, le galactose, le mannose, la N-acétylglucosamine ou le fucose.

12. Utilisation selon la revendication 9, dans laquelle X est le ribose.

13. Utilisation selon la revendication 9, dans laquelle le dérivé de nucléoside est le 5'-adénosine-diphosphate ribose.

14. Utilisation selon l'une quelconque des revendications 9 à 13, dans laquelle la composition est dans la forme d'une pastille orale.

15. Utilisation selon l'une quelconque des revendications 9-13, dans laquelle la composition est une composition orale, de préférence une eau de bouche.

16. Utilisation selon la revendication 15, dans laquelle l'eau de bouche comprend du 5'-adénosine-diphosphate ribose à une concentration d'au moins 10 µg/ml, de préférence de 10 µg/ml à 1000 µg/ml.

17. Utilisation selon l'une quelconque des revendications 9-13, dans laquelle la composition est un liquide nutritionnel oral comprenant de plus une protéine, un carbohydrate et une graisse.

18. Composition orale comprenant une quantité thérapeutiquement efficace de 5'-adénosine-diphosphate ribose pour une utilisation dans le traitement d'une inflammation d'une muqueuse.
